# EUROPÄISCHE PATENTSCHRIFT

(11) **EP 2 310 104 B1**
(45) Veröffentlichungstag und Bekanntmachung des Hinweises auf die Patenterteilung: **18.10.2017**
(21) Anmeldenummer: 09802444.1
(22) Anmeldetag: 08.07.2009
(51) Int. Cl.: B01D 15/36, B01J 39/26, B01J 41/20, C07K 1/18

(54) **MISCHPFROPFPOLYMERE FÜR DIE IONENAUSTAUSCHCHROMATOGRAPHIE**
MIXED GRAFT POLYMERS FOR ION EXCHANGE CHROMATOGRAPHY
POLYMÈRE GREFFÉ MIXTE POUR LA CHROMATOGRAPHIE À ÉCHANGE IONIQUE

(30) Priorität: 30.07.2008 EP 08013674
(43) Veröffentlichungstag der Anmeldung: 20.04.2011
(73) Patentinhaber: Merck Patent GmbH, 64293 Darmstadt (DE)
(72) Erfinder: GRAALFS, Heiner, 64372 Ober-Ramstadt (DE); BRITSCH, Lothar, 79276 Reute (DE)
(86) Internationale Anmeldenummer: PCT/EP2009/004952
(87) Internationale Veröffentlichungsnummer: WO 2010/012358

(56) Entgegenhaltungen:
- EP-A- 1 473 075
- EP-A1- 0 337 144

## Beschreibung

Die Erfindung betrifft ein modifiziertes Trennmaterial, dessen Herstellung und dessen Anwendung zur Abtrennung von geladenen Biopolymeren aus Flüssigkeiten.

### Stand der Technik

Die Ionenaustauschchromatographie ist die wohl am häufigsten eingesetzte Methode zur Reinigung von pharmazeutischen Proteinen, Peptiden und anderen Biopolymeren. Nahezu alle industriellen Reinigungsprozesse beinhalten einen oder mehrere lonenaustauschschritte. Von besonderem Interesse sind lonenaustauscherharze, die eine hohe Bindungskapazität für die Zielmolekühle besitzen.

Besonders vorteilhaft ist die Verwendung von geeigneten Trennmaterialien, bei denen die ionische Gruppen ausschließlich auf linearen Pfropfpolymeren lokalisiert sind, welche wiederum kovalent an eine Trägeroberfläche gebunden sind (W. Müller, J. Chromatography 1990, 510, 133-140).

Funktionalisierte Polymere, die durch Pfropfung entsprechender funktionalisierter Monomere auf eine Vielzahl unterschiedlicher Oberflächen erhalten werden, sind seit vielen Jahren bekannt. Wenn es sich bei der Funktionalisierung um chemisch gebundene ionische Gruppierungen handelt, können entsprechende Materialien für die Ionenaustauschchromatographie verwendet werden.

Eine größere Anzahl an möglichen Pfropfpolymerstrukturen, die für die Fraktionierung von Biopolymeren vorgesehen sind, findet sich in den Patenten EP 0 337 144 oder US 5,453,186. Auch Pfropfpolymere aus mehr als einem Monomerbaustein (Copolymerisation) sind in den Patenten beansprucht. Für diese Mischpfropfpolymerisation müssen dabei, um geeignete Austauscher zu erhalten, die Monomere so gewählt werden, dass beide Monomere entweder basische oder saure Gruppen enthalten oder ein Monomer neutral ist. Welche Kombination und welche Art von Monomeren für die Mischpfropfpolymerisation zu bevorzugen ist, wird nicht erläutert, so dass der Fachmann, bevor er für die Reinigung von pharmazeutischen Proteinen, Peptiden und anderen Biopolymeren geeignete Trennmaterialien erhält, eine große Anzahl von Versuchen durchführen muss.

Durch EP 1 473 075 A werden Materialien für die lonenaustauscherchromatographie beschrieben. Zur Herstellung der Materialien wird eine Polymerlösung, bestehend aus 2-Acrylamido-2-methyl-1-propansulfonsäure, N(Isobutoxymethyl)acrylamid und Hydroxymethylmethacrylat auf hydroxylgruppenhaltige Träger, wie z. B. poröses Cellulosenitrat, aufgezogen. Es erfolgt jedoch durch dieses "Aufziehen" keine chemische Verknüpfung zwischen dem Trägermaterial und dem aufgebrachten Coating. Bei dem Aufziehen wird ein Hydrogel, wobei es sich um ein in Wasser gequollenes, vernetztes Polymer handelt, in den Poren des Trägers polymerisiert. Erst durch das Polymerisieren in den Poren wird das Material ausreichend formstabil, so dass es in der Anwendung druckstabil ist. Zur Herstellung des verwendeten Hydrogels sind mindestens drei Monomere, erforderlich; und für die Herstellung des eigentlichen Trennmaterials sind mindestens fünf Komponenten nämlich Träger, Monomer 1, Monomer 2 und Vernetzer und Dextran erforderlich.

Kobayashi et al. [Journal of Chromatography, Bd.958, Nr. 1-2, 109 - 119, (2002)] wiederum beschreiben ein Chromatographiematerial, welches durch Aufpfropfen eines Polymers aus Isopropylacrylamid und Acrylsäure auf Silica, bzw. Kieselgel erhalten wird. Ziel dieser Aufpfopfung ist es, durch Einbringen eines ungeladenen Monomers hydrophobe Eigenschaften zu erzeugen. Eine Bindung der Polymere an das anorganische Silica ist dadurch erschwert, dass keine aliphatischen Hydroxylgruppen an der trägeroberfläche vorhanden sind und Silica hydrolysestabil ist.

Durch US 2004/203149 A1 wird chromatographisches Material beschrieben, das aus einem Trägermaterial und einem Gel besteht. Es werden trägergestützte poröse, quervernetzte Gele erhalten. Wie auch in EP 1 473 075 A beschreiben, wird eine Hydrogel in den Poren des Trägers polymerisiert, woduurch ein ausreichend formstabiles Material erhalten wird, das in der Anwendung druckstabil ist. Es erfolgt keine kovalente Bindung an den Träger, vielmehr wird das Hydrogel mit dem Träger verflochten. Bevorzugt werden hydrophobe Trägermaterialien verwendet. Wie auch durch Kobayashi beschrieben, ist zur Herstellung des gewünschten Trennmaterials in jedem Fall ein Vernetzer erforderlich, der in der Anwendung der Materialien einen erheblichen Einfluss auf das Bindeverhalten hat. Darüber hinaus ist das bevorzugt verwendete Acrylamid nicht ausreichend hydrolysestabil.

### Aufgabe

Aufgabe der vorliegenden Erfindung ist es daher, ein Trägermaterial mit ionischen Gruppen herzustellen, das Proteine und andere Biopolymere mit hoher Kapazität bindet, und welches für präparative Anwendungen im industriellen Maßstab einsetzbar ist.

Dieses Trägermaterial soll hydrolysestabil, insbesondere alkalistabil sein, um eine Reinigung bzw. Regeneration des Trennmaterials bei ph ≥ 13 zu ermöglichen, wobei wesentliche Eigenschaften des Trägers erhalten bleiben.

### Gegenstand der Erfindung

Gegenstand der Erfindung sind Trennmaterialien, enthaltend anorganische oder organische Trägermaterialien, dadurch gekennzeichnet, dass an das Trägermaterial Polymere, aufgebaut aus mindestens zwei Monomereinheiten gebunden sind, wobei mindestens 2 verschiedene Wiederholungseinheiten in dem Trennmaterial vorkommen, während eine dieser Einheiten eine Ladung führt und mindestens eine dieser Einheiten eine neutrale Gruppe trägt.

Durch Versuche wurde gefunden, dass mit N-Alkoxyalkylacrylamiden als neutrales Comonomer derivatisierte Trägermaterialien als Trennmaterialien geeignet sind für die Abtrennung von geladenen Biomolekülen, insbesondere von geladenen Biopolymeren. Es wird durch die Mischpfropfpolymerisation mit N-Alkoxyalkylacrylamiden eine gute Ausnutzung von kleinen Ladungsdichten auf der Oberfläche erreicht.

Die Lösung der vorliegenden Aufgabe besteht daher auch in der Herstellung von entsprechenden Mischpfropfpolymeren auf hydroxylgruppenhaltigen Oberflächen von porösen Partikeln oder sonstigen Formkörpern. Die durch die Reaktion an diesen Oberflächen gebundenen Pfropfpolymere sind erfindungsgemäß aus zwei oder mehreren wiederkehrenden Einheiten aufgebaut, wobei mindestens eine dieser Einheiten eine Ladung trägt und mindestens eine Einheit mit einer neutralen Gruppe verknüpft ist, so dass diese Pfropfpolymere durch ionische Wechselwirkung eine geladene Substanz zu binden vermögen.

Gegenstand der vorliegenden Erfindung sind daher weiter Trennmaterialien, welche durch ionische Wechselwirkung geladene Substanzen, insbesondere Biopolymere, zu binden vermögen. Zur Herstellung dieser Trägermaterialien können durch chemische Reaktion Pfropfpolymere, aufgebaut aus zwei oder mehreren wiederkehrenden Monomereinheiten, wobei mindestens eine dieser Einheiten eine Ladung trägt und mindestens eine dieser Einheiten mit einer neutralen Gruppe verknüpft ist, an hydroxylgruppenhaltige Oberflächen poröser Partikel oder sonstiger Formkörper gebunden werden.

Gegenstand der vorliegenden Erfindung sind insbesondere entsprechende Trennmaterialien, die ein quervernetztes synthetisches Polymer oder eine quervernetzte Agarose oder ein Dextran oder ein Kompositmaterial aus anorganischem Träger mit organischem Coating als Basisträger aufweisen.

Solche Trennmaterialien für die Ionenaustauschchromatographie auf der Grundlage entsprechender hydroxylgruppenhaltiger Basisträger, auf deren Oberflächen Mischpolymere kovalent gebunden sind, zeichnen sich dadurch aus, dass sie ein quervernetztes synthetisches Polymer oder eine quervernetzte Agarose oder ein quervernetztes Dextran oder ein Kompositmaterial aus anorganischem Träger mit organischem Coating als Trägermaterial enthalten, auf dessen Oberfläche Mischpolymere gebunden sind und dass
a) der Basisträger aliphatische Hydroxylgruppen enthält,
b) die Mischpolymeren kovalent an den Träger gebunden sind,
c) die Mischpolymeren mindestens zwei verschiedene Monomereinheiten enthalten
d) die Monomereinheiten linear verknüpft sind,
e) das Mischpolymer mindestens eine Monomereinheit aufweist, die eine Ladung in Form einer Sulfonsäure oder Carbonsäure trägt, oder eine Amin- oder Ammoniumgruppe trägt und daneben und Alkyl- und/oder Alkylengruppen und gegebenenfalls Amidgruppen enthält, jedoch keine Arylgruppen aufweist,
f) das Mischpolymer mindestens eine ungeladene Monomereinheit der allgemeinen Formel (1) 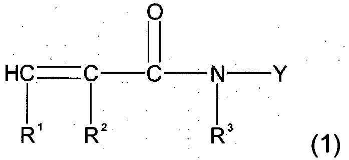 aufweist,
   worin
   R¹ Wasserstoff,
   R² Wasserstoff oder Methyl, und
   R³ und Y unabhängig voneinander Wasserstoff, geradkettiges Alkyl mit bis zu 4 C-Atomen, Methoxypropyl, Ethoxyethyl oder Methoxyethyl bedeuten,
g) nur Monomereinheiten gleicher Ladung kombiniert sind, wenn mehr als ein Monomer mit Ladung enthalten ist,
h) das Verhältnis der Monomereinheiten mit Ladung zu den Monomereinheiten ohne Ladung in einem Bereich zwischen 1 : 99 bis 90 : 10 liegt.

Gegenstand dervorliegenden Erfindung sind bevorzugt auch entsprechende Trennmaterialien für die Ionenaustauschchromatographie auf der Grundlage von hydroxylgruppenhaltigen Basisträgern, auf deren Oberflächen Mischpolymere kovalent gebunden sind, und welche dadurch gekennzeichnet sind, dass
a) der Basisträger aliphatische Hydroxylgruppen enthält,
b) die Mischpolymere kovalent an den Träger gebunden sind,
c) die Mischpolymere mindestens zwei verschiedene Monomereinheiten enthalten
d) die Monomereinheiten linear verknüpft sind,
e) das Mischpolymer mindestens eine Monomereinheit mit Ladung der allgemeinen Formel (1) aufweist, worin
   - R¹: Wasserstoff,
   - Y: Wasserstoff und
   - R³: R⁴-SO₃Z, R⁴-COO, R⁴-NR⁹R¹⁰ oder R⁴-NR⁹R¹⁰R¹¹X
   mit
   R⁴ geradkettiges oder verzweigtes Alkylen mit 2 bis 4 C-Atomen,
   R⁹, R¹⁰ und R¹¹ unabhängig von einander Wasserstoff, Methyl, Ethyl, Propyl, Phenyl oder Benzyl,
   Z Wasserstoff, Na, K oder NH₄ und
   X Cl, Br, J, oder Methylsulfat
   bedeuten
   oder das Mischpolymer mindestens eine Monomereinheit der allgemeinen Formel (2) 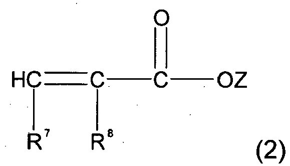 aufweist, worin
   - R⁷: Wasserstoff,
   - R⁸: Methyl,
   - Z: Wasserstoff, Na, K, oder NH₄
   bedeuten.
   Bevorzugt sind erfindungsgemäße Trennmaterialien dadurch gekennzeichnet, dass
f) das gebundene Mischpolymer mindestens eine ungeladene Monomereinheit der allgemeinen Formel (1) 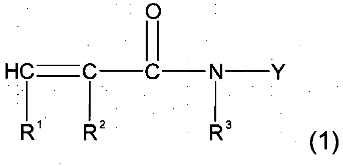 aufweist,
   worin
   - R¹: Wasserstoff, R² Wasserstoff oder Methyl und R³ Methyl, Ethyl, Propyl, Methoxypropyl, Methoxyethyl mit Y Wasserstoff oder R³ Methyl oder Ethyl mit Y Methyl bedeuten.

Eine besonders bevorzugte Variante der erfindungsgemäßen Trennmaterialien zeichnet sich dadurch aus, dass
c) die Mischpolymere aus mindestens zwei verschiedenen Monomereinheiten aufgebaut sind, und
e) das Mischpolymer mindestens eine Monomereinheit mit negativer Ladung ausgewählt aus der Gruppe 2-Acrylamido-2-methylpropan-sulfonsäure, 2-Acrylamidoethansulfonsäure, Carboxymethylacrylamid; Carboxyethylacrylamid, Carboxypropylacrylamid, Carboxymethlymethacrylamid, Carboxyethlymethacrylamid, Carboxypropylmethacrylamid, Acrylsäure und Methacrylsäure, aufweist
   und
f) das Mischpolymer mindestens eine ungeladene Monomereinheit gemäß der allgemeinen Formel (1), ausgewählt aus der Gruppe N,N-(Dimethyl)acrylamid, N-(Ethoxyethyl)acrylamid, N-(Methoxyethyl)acrylamid und N-(Methoxypropyl)acrylamid, enthält.

Eine weitere bevorzugte Ausführungsform der erfindungsgemäß beschriebenen Trennmaterialien zeichnet sich dadurch aus, dass
c) die Mischpolymere mindestens zwei verschiedene Monomereinheiten enthalten,
e) die Mischpolymere mindestens eine Monomereinheit mit positiver Ladung, ausgewählt aus der Gruppe 2-(Acryloylaminoethyl)trimethylammoniumchlorid, 3-(Acryloylaminopropyl)-trimethylammoniumchlorid, 2-(Diethylaminoethyl)acrylamid, 2-(Diethylaminoethyl)methacrylamid, 2-(Dimethylaminoethyl)acrylamid, 2-(Dimethylaminoethyl)-methacrylamid, 3-(Diethylaminopropyl)acrylamid, 3-(Diethylaminopropyl)methacrylamid, 3-(Diethylaminopropyl)acrylamid, 3-(Diethylaminopropyl)methacrylamid, 2-(Methacryloylaminoethyl)-trimethylammoniumchlorid und 3-(Acryloyl-aminopropyl)-trimethylammoniumchlorid aufweisen und
f) die Mischpolymere mindestens eine ungeladene Monomereinheit gemäß der allgemienen Formel (1), ausgewählt aus der Gruppe N,N-(Dimethyl)acrylamid, N-(Ethoxyethyl)acrylamid, N-(Methoxyethyl)acrylamid und N-(Methoxypropyl)acrylamid, enthalten.

Gegenstand der vorliegenden Erfindung ist darüber hinaus ein Verfahren zur Herstellung der so charakterisierten Trennmaterialien, welches dadurch charakterisiert ist, dass Mischpolymere an Oberflächen eines Basisträgers mit aliphatischen Hydroxylgruppen,
wobei es sich um Oberflächen von einem quervernetzten synthetischen Polymer oder einer quervernetzten Agarose oder einem quervernetzten Dextran oder einem Kompositmaterial aus anorganischem Träger mit organischem Coating handelt,
als Trägermaterial gebunden werden,
indem
durch eine einstufige Polymerisationsreaktion,
welche durch Cer(IV)-Ionen auf dem hydroxylgruppen-haltigen Träger initiiert wird,
Pfropfpolymere erhalten werden,
die kovalent an die hydroxylgruppenhaltige Oberfläche gebunden sind,
und
die aus zwei oder mehreren wiederkehrenden Monomereinheiten aufgebaut sind und die linear verknüpft sind,
wobei mindestens eine dieser Monomereinheiten eine Ladung
in Form einer Sulfonsäure oder einer Carbonsäure trägt, oder
eine Amin- oder Ammoniumgruppe trägt und Alkyl- und/oder Alkylengruppen und gegebenenfalls Amidgruppen enthält, jedoch keine Arylgruppen aufweist,
und mindestens eine dieser wiederkehrenden Monomereinheiten ungeladen ist und die allgemeinen Formel (1) 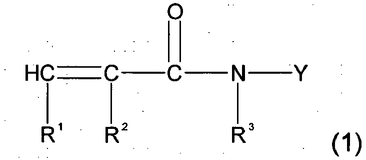 aufweist, worin
R¹ Wasserstoff,
R² Wasserstoff oder Methyl, und
R³ und Y unabhängig voneinander Wasserstoff, geradkettiges Alkyl mit bis zu 4 C-Atomen, Methoxypropyl, Ethoxyethyl oder Methoxyethyl bedeuten,
wobei nur Monomereinheiten gleicher Ladung kombiniert sind, wenn mehr als ein Monomer mit Ladung im Pfropfpolymer enthalten ist,
und das Verhältnis der Monomereinheiten mit Ladung zu den Monomereinheiten ohne Ladung in einem Bereich zwischen
1 : 99 bis 90 : 10 liegt,
wodurch Materialien erhalten werden, die durch ionische Wechselwirkung geladene Substanzen, insbesondere Biopolymere, zu binden vermögen.

### Detaillierte Beschreibung der Erfindung

Die Verwendung von flexiblen, an die Trägeroberfläche gebundenen Pfropfpolymeren ("Tentakeln") als ionenaustauschende Gruppe ist bekannt. So ist in dem kommerziell erhältlichen Kationenaustauscher Fractogel^{®} EMD SO₃⁻ (M) das Pfropfpolymer nur aus einer wiederkehrenden Einheit mit Sulfonsäuregruppen aufgebaut.

In den Patenten EP 0 337 144 oder US 5,453,186 sind keine Informationen zu Monomerkombinationen aufgeführt, die bei der Synthese eines lonenaustauschers zu bevorzugen sind. Auch in den Beispielen sind nur Pfropfpolymerisationen mit einem Monomer beschrieben, die direkt zu einem lonenaustauscher führen.

Insbesondere die Pfropfung von funktionalisierten Acrylamiden und Acrylsäure wurde untersucht, da die daraus gebildeten Polymere unter alkalischen Bedingungen hydrolysestabil sind. Außerdem können die Poly(acrylamide) Wasserstoffbrückenbindungen ausbilden, was für die Anwendung auf Grund der besseren Quellbarkeit in wässrigen Lösungen und damit ihrer Hydrophilie ein großer Vorteil ist.

Zur Herstellung der erfindungsgemäßen Trennmaterialien können hydrophile Chromatographieträger, wie z. B. Fractogel TSK HW65 (M) oder das kommerziell erhältlichen Toyopearl HW-65 (S), verwendet werden. Diese Träger werden mit Mischpfropfpolymeren modifiziert.

Für die Herstellung der erfindungsgemäßen Materialien können auch andere Chromatographieträger verwendet werden. Bevorzugt ist jedoch, wenn das verwendete Material für die Pfropfpolymerisationsreaktion zugängliche reaktive Gruppen, bevorzugt OH-Gruppen aufweist. Geeignete Trägermaterialien können daher zum Beispiel auch aus organischen Polymeren bestehen. Solche organischen Polymere können Polysaccharide, wie Agarose, Dextrane, Stärke, Zellulose, usw., oder synthetische Polymere, wie Poly(acrylamide), Poly(methacrylamide), Poly(acrylate), Poly(methacrylate), hydrophil substituierte Poly(alkylallylether), hydrophil substituierte Poly(alkylvinylether), Poly(vinylalkohole), Poly(n-vinylharnstoffe), Poly(N-vinylpyrrolidone), Poly(styrrole) und Copolymere der korrespondierenden Monomere, sein. Diese organischen Polymere können bevorzugt auch in Form eines quervernetzten hydrophilen Netzwerks eingesetzt werden. Zu diesen quervernetzten hydrophilen Polymeren zählen auch solche aus Styrrol und Divinylbenzol, die bevorzugt, wie andere hydrophobe Polymere auch, in einer hydrophilisierten Form eingesetzt werden können.

Gleichermaßen können Kompositmaterialien als geeignete Trägermaterialien eingesetzt werden, d.h. es können beispielsweise erfindungsgemäße Trennmaterialien durch Derivatisierungen der Oberfläche z.B. von anorganischen Partikeln oder Formkörpern erhalten werden, die in erfindungsgemäßer Weise derivatisiert werden. Ein Beispiel hierfür sind Partikel, die durch Einpolymerisierung magnetisierbarer Teilchen oder eines magnetisierbaren Kerns selbst magnetisierbar sind.

Bevorzugt werden jedoch hydrophile Trägermaterialien eingesetzt, die stabil gegenüber Hydrolyse sind oder sich nur schwer hydrolysieren lassen, weil die erfindungsgemäßen Materialien einer alkalischen Reinigung bzw. Regeneration bei ph ≥ 13 über eine längere Anwendungsdauer standhalten müssen. Die Träger können bereits niedermolekulare Liganden tragen. Liganden können ein oder mehrere geladene Gruppen, hydrophobe Gruppen oder Gruppen, die Wasserstoffbrückenbindungen bilden können, tragen. Bevorzugt sind Liganden, die die gleiche Ladung tragen wie das Pfropfpolymer.

Die Trägermaterialien können auch aus unregelmäßig geformten oder aus sphärischen Partikeln bestehen, deren Partikelgröße zwischen 2 und 1000 µm liegen kann. Bevorzugt sind Partikelgrößen zwischen 3 und 300 µm.

Die Trägermaterialien können insbesondere als unporöse oder bevorzugt als poröse Partikel vorliegen. Die Porengrößen können zwischen 2 und 300 nm liegen. Bevorzugt sind Porengrößen zwischen 5 und 200 nm.

Genauso können die Trägermaterialien auch in Form von Membranen, Fasern, Hohlfasern, Beschichtungen oder als monolithische Formkörper vorliegen. Monolithische Formkörper sind dreidimensionale Körper, z. B. in zylindrischer Form.

Für die bevorzugte Pfropfpolymerisation wird mindestens ein positiv oder negativ geladenes Monomer verwendet. Falls mehrere geladene Monomere eingesetzt werden, dürfen nur gleich geladene Monomere gemischt werden, um Trennmaterialien mit erfindungsgemäß verbesserten Eigenschaften zu erhalten. Monomere mit negativer Gruppe können beispielsweise Sulfonsäure- oder Carboxy-Gruppen aufweisen.

Geeignete Monomere mit Sulfonsäure-Gruppen sind beispielsweise Acrylate der Formel (2) mit Z = R⁴-SO₃M, worin R⁷ und R⁸ unabhängig voneinander die Bedeutungen Wasserstoff oder Alkyl mit bis zu 6 C-Atomen, bevorzugt Wasserstoff oder Methyl, Carboxy oder Carboxymethyl haben können und worin R⁴ eine geradkettige Alkylengruppe mit 1 bis 8 C-Atomen, wie zum Beispiel Methylen, Ethylen, Propylen oder Hexylen, oder eine verzweigte Alkylengruppe mit 1 bis 8 C-Atomen sein kann, wie zum Beispiel Isopropylene oder Isobutylen.

M ist ein Wasserstoffatom oder ein Metallkation, wie Natrium oder Kalium, oder ein Ammoniumkation. M ist so gewählt, dass das Monomer wasserlöslich ist.

Beispielhaft sind die Sulfoalkylacrylate, wie 3-Sulfopropylacrylat oder 2-Sulfoethylacrylat, und die Sulfoalkylmethacrylate, wie 3-Sulfopropylmethacrylat oder 2-Sulfoethylmethacrylat erwähnt.

Bevorzugt werden die Acrylamide der allgemeinen Formel (1) 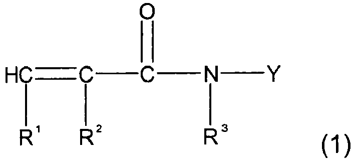 mit R³ = R⁴-SO₃M eingesetzt, worin R¹, R² und Y unabhängig voneinander die Bedeutungen Wasserstoff oder Alkyl mit bis zu 6 C-Atomen haben, bevorzugt Wasserstoff oder Methyl sein können. R¹ und R² können ebenfalls unabhängig voneinander Carboxy oder Carboxymethyl sein.

R³ kann auch R⁴-SO₃M sein, und worin R⁴ eine geradkettige Alkylengruppe mit 1 bis 8 C-Atomen, wie zum Beispiel Methylen, Ethylen, Propylen oder Hexylen, oder eine verzweigte Alkylengruppe mit 1 bis 8 C-Atomen sein kann, wie zum Beispiel Isopropylene oder Isobutylen.

M ist ein Wasserstoffatom oder ein Metallkation, wie Natrium oder Kalium, oder ein Ammoniumkation. M ist so gewählt, dass das Monomer wasserlöslich ist.

Als geeignete Acrylamide seien hier beispielhaft 2-Acrylamido-2-methylpropansulfonsäure (AMPS) und 2-Acrylamidoethansulfonsäure genannt.

Geeignete Monomere mit Carboxy-Gruppe können beispielsweise auch Acrylate der allgemeinen Formel (2) 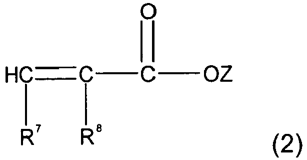 mit Z = R⁵-COOM sein, worin R¹ und R² unabhängig voneinander die Bedeutungen Wasserstoff oder Alkyl mit bis zu 6 C-Atomen, bevorzugt Wasserstoff oder Methyl, Carboxy oder Carboxymethyl haben können, und worin R⁵ eine geradkettige Alkylengruppe mit 1 bis 8 C-Atomen, wie zum Beispiel Methylen, Ethylen, Propylen oder Hexylen, oder eine verzweigte Alkylengruppe mit 1 bis 8 C-Atomen sein kann, wie zum Beispiel Isopropylene oder Isobutylen.

M ist ein Wasserstoffatom oder ein Metallkation, wie Natrium oder Kalium, oder ein Ammoniumkation. M ist so gewählt, dass das Monomer wasserlöslich ist.

Beispielhaft sind die Carboxyalkylacrylate, wie Carboxyethylacrylat und die Carboxyalkylmethacrylate erwähnt. Bevorzugt werden die Acrylamide der Formel (1) mit R³ = R⁵-COOM eingesetzt, worin R¹, R² und Y unabhängig voneinander die Bedeutungen Wasserstoff oder Alkyl mit bis zu 6 C-Atomen haben, bevorzugt Wasserstoff oder Methyl, R¹ und R² können unabhängig voneinander ebenfalls Carboxy oder Carboxymethyl sein.

R³ kann auch R⁵-COOM sein, und worin R⁵ eine geradkettige Alkylengruppe mit 1 bis 8 C-Atomen, wie zum Beispiel Methylen, Ethylen, Propylen oder Hexylen, oder eine verzweigte Alkylengruppe mit 1 bis 8 C-Atomen sein kann, wie zum Beispiel Isopropylene oder Isobutylen.

M ist ein Wasserstoffatom oder ein Metallkation, wie Natrium oder Kalium, oder ein Ammoniumkation. M ist so gewählt, dass das Monomer wasserlöslich ist.

Besonders bevorzugt werden ungesättigte Carbonsäuren der allgemeinen Formel (2) mit Z = M eingesetzt, worin R⁷ und R⁸ unabhängig voneinander die Bedeutungen Wasserstoff oder Alkyl mit bis zu 6 C-Atomen, bevorzugt Wasserstoff oder Methyl, Carboxy oder Carboxymethyl haben können. M ist ein Wasserstoffatom oder ein Metallkation, wie Natrium oder Kalium, oder ein Ammoniumkation. M ist so gewählt, dass das Monomer wasserlöslich ist. Beispielhaft seien Maleinsäure, Itaconsäure, Citraconsäure, Mesaconsäure, oder Fumarsäure genannt. Davon werden insbesondere Monomere der Formel (2) mit Z = M bevorzugt, worin R⁷ Wasserstoff und R8 Wasserstoff oder Alkyl mit bis zu 3 C-Atomen bedeuten. Beispielhaft seien hierfür Acrylsäure und Methacrylsäure genannt.

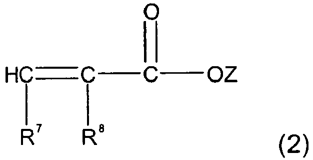

Monomere mit positiver Gruppe können beispielsweise primäre, sekundäre oder tertiäre Aminogruppen tragen oder quartäre Ammoniumsalze sein. Geeignete Monomere mit Amino-Gruppen sind beispielsweise Acrylate der Formel (2) mit Z = R⁴-NR⁹R¹⁰, worin R⁷ und R⁸ unabhängig voneinander die Bedeutungen Wasserstoff oder Alkyl mit bis zu 6 C-Atomen, bevorzugt Wasserstoff oder Methyl haben können und worin R⁴ eine geradkettige Alkylengruppe mit 1 bis 8 C-Atomen, wie zum Beispiel Methylen, Ethylen, Propylen oder Hexylen, oder eine verzweigte Alkylengruppe mit 1 bis 8 C-Atomen sein kann, wie zum Beispiel Isopropylene oder Isobutylen, und worin R⁹ und R¹⁰ unabhängig voneinander die Bedeutungen Wasserstoff, Alkyl, Phenyl oder Alkylphenyl, wie zum Beispiel Methyl, Ethyl oder Benzyl haben. Beispielhaft sind die Aminoalkylacrylate, wie 2-(Diethylaminoethyl)acrylat, 2-(Dimethylaminoethyl)acrylat oder 2-(Dimethylaminopropyl)acrylat, und die Aminoalkylmethacrylate, wie 2-(Diethylaminoethyl)methacrylat, 2- (Dimethylaminoethyl)methacrylat oder 3-(Diethylaminopropyl)methacrylat erwähnt.

Bevorzugt werden die Acrylamide der Formel (1) mit R³ = R⁴- R⁴-NR⁹R¹⁰ eingesetzt, worin R¹, R² und Y unabhängig voneinander die Bedeutungen Wasserstoff oder Alkyl mit bis zu 6 C-Atomen haben, bevorzugt Wasserstoff oder Methyl, und worin R4 eine geradkettige Alkylengruppe mit 1 bis 8 C-Atomen, wie zum Beispiel Methylen, Ethylen, Propylen oder Hexylen, oder eine verzweigte Alkylengruppe mit 1 bis 8 C-Atomen sein kann, wie zum Beispiel Isopropylene oder Isobutylen, und worin R⁹ und R¹⁰ unabhängig voneinander die Bedeutungen Wasserstoff, Alkyl, Phenyl oder Alkylphenyl, wie zum Beispiel Methyl, Ethyl oder Benzyl haben. Als geeignete Acrylamide seien hier beispielhaft 2-(Diethylaminoethyl)acrylamid, 2-(Dimethylaminoethyl)acrylamid, 3-(Diethylaminopropyl)acrylamid oder 3-(Diethylaminopropyl)acrylamid genannt und als geeignete Methacrylamide seien hier beispielhaft 2-(Diethylaminoethyl)methacrylamid, 2-(Dimethylaminoethyl)-methacrylamid, 3-(Diethylaminopropyl)methacrylamid oder 3-(Diethylaminopropyl)methacryl-amid genannt.

Geeignete Monomere, die quartäre Ammoniumsalze sind, sind beispielsweise Acrylate der allgemeinen Formel (2), worin Z die Bedeutung R⁴-NR⁹R¹⁰R¹¹X hat, und worin R⁷ und R⁸ unabhängig voneinander die Bedeutungen Wasserstoff oder Alkyl mit bis zu 6 C-Atomen, bevorzugt Wasserstoff oder Methyl, haben können, und worin R4 eine geradkettige Alkylengruppe mit 1 bis 8 C-Atomen, wie zum Beispiel Methylen, Ethylen, Propylen oder Hexylen, oder eine verzweigte Alkylengruppe mit 1 bis 8 C-Atomen sein kann, wie zum Beispiel Isopropylene oder Isobutylen, und worin R⁹, R¹⁰ und R¹¹ unabhängig voneinander die Bedeutungen Wasserstoff, Alkyl, Phenyl oder Alkylphenyl, wie zum Beispiel Methyl, Ethyl oder Benzyl haben. X ist ein Anion und so gewählt, dass das Monomer wasserlöslich ist, und kann zum Beispiel Chlorid, Bromid, Iodid oder Methylsulfat sein. Beispielhaft sind die Acryloxyalkylammoniumsalze, wie [2-(Acryloxy)ethyl]-trimethylammonium-chlorid und Methacryloxyalkylammoniumsalze, wie [2-(Methacryloxy)-ethyl]trimethylammoniumchlorid erwähnt.

Bevorzugt werden die Acrylamide der allgemeinen Formel (1) mit R³ = R⁴-NR⁹R¹⁰R¹¹X eingesetzt, worin R¹, R² und Y unabhängig voneinander die Bedeutungen Wasserstoff oder Alkyl mit bis zu 6 C-Atomen haben, bevorzugt Wasserstoff oder Methyl, und worin R⁴ eine geradkettige Alkylengruppe mit 1 bis 8 C-Atomen, wie zum Beispiel Methylen, Ethylen, Propylen oder Hexylen, oder eine verzweigte Alkylengruppe mit 1 bis 8 C-Atomen sein kann, wie zum Beispiel Isopropylene oder Isobutylen, und worin R⁹, R¹⁰ und R¹¹ unabhängig voneinander die Bedeutungen Wasserstoff, Alkyl, Phenyl oder Alkylphenyl, wie zum Beispiel Methyl, Ethyl oder Benzyl haben. X ist ein Anion und so gewählt, dass das Monomer wasserlöslich ist, und kann zum Beispiel Chlorid, Bromid, Iodid oder Methylsulfat sein. Als geeignete Acrylamide seien hier beispielhaft 2-(Acryloylaminoethyl)-trimethylammonium-chlorid und 3-(Acryloylaminopropyl)trimethylammoniumchlorid genannt. Als geeignete Methacrylamide seien hier beispielhaft 2-(Methacryloyl-aminoethyl)-trimethyl-ammoniumchlorid und 3-(Acryloylamino-propyl)-trimethylammoniumchlorid genannt.

Als weitere Komponente wird mindestens ein ungeladenes Monomer benötigt, das vorzugsweise hydrophil ist. Für diesen Zweck geeignete neutrale Monomere sind beispielsweise niedere Alkylacrylate, wie Methylacrylat, niedere Alkylmethacrylate, wie Methylmethacrylat. Bevorzugt werden Acrylamide der allgemeinen Formel 1 mit Y = R⁶ eingesetzt, worin R⁷ und R² unabhängig voneinander Wasserstoff oder Methyl sind und worin R³ und R⁶ unabhängig voneinander Wasserstoff oder Alkyl mit bis zu 4 C-Atomen bedeuten. R³ und/oder R⁶ bedeuten damit Wasserstoff oder niederes Alkyl. Letzteres hat hier bevorzugt die Bedeutung Methyl, Ethyl, Butyl, Isopropyl, 3-Butyl oder Isobutyl sowie außerdem die Bedeutung von Alkoxyalkyl mit bis zu 4 C-Atomen, wie z. B. Methoxyethyl oder Ethoxyethyl. Beispielhaft seien hier Acrylamid (AAm), Dimethyl-acrylamid, Ethoxyethylacrylamid, Methacrylamid, Methoxyethylacrylamid und Methoxypropylacrylamid genannt. Bevorzugt sind die N-substituierten Amide, da sie hydrolysestabiler sind als Ester und unsubstituierte Amide.

Das eigentliche Polymer kann auf verschiedene Weisen hergestellt werden. Beim "grafting onto" müssen aus den Monomeren zuerst Polymerketten hergestellt werden, die in einem zweiten Schritt an die Oberfläche gebunden werden. Beim "grafting from" wird eine Polymerisationsreaktion an der Oberfläche gestartet und das Pfropfpolymer direkt aus einzelnen Monomeren aufgebaut. Auch andere Polymerisationsmethoden, die eine Anbindung an die Oberfläche des Trägermaterials erlauben, können eingesetzt werden. Erfindungsgemäss ist die zweite Methode und besonders bevorzugt werden Varianten, bei denen nur wenige Nebenprodukte, wie nicht kovalent gebundenes Polymer, entstehen, die abgetrennt werden müssen. Beschrieben werden Verfahren mit kontrollierter Radikalpolymerisation, wie zum Beispiel die Methode der Atomtransfer-Radikalpolymerisation (ATRP). Hier wird in einer ersten Stufe eine Initiatorgruppe in der gewünschten Dichte kovalent an die Trägeroberfläche gebunden. Eine Inititiatorgruppe kann zum Beispiel ein über eine Esterfunktion gebundenes Halogenid sein, wie in einem 2-Brom-2-methylpropionsäureester. Die Pfropfpolymerisation erfolgt in einer zweiten Stufe in Anwesenheit von Kupfer(I)-Salzen. Die erfindungsgemässe einstufige Pfropfpolymerisationsreaktion wird durch Cer(IV) auf dem hydroxylgruppen-haltigen Träger initiiert ohne dass der Träger aktiviert werden muss. Diese Reaktion findet normalerweise in verdünnten Mineralsäuren statt. Üblicherweise wird zur Durchführung dieser Pfropfpolymerisation die Säure in einer wässrigen Lösung mit einer Konzentration im Bereich von 1 bis 0,00001 mol/l, bevorzugt von 0,1 bis 0,001, eingesetzt. Ganz besonders bevorzugt wird mit verdünnter Salpetersäure gearbeitet, die mit einer Konzentration im Bereich von 0,1 bis 0,001 mol/l eingesetzt wird.

Für Versuchsreihen zur Pfropfung wurden bevorzugt funktionalisierte Acrylamide und Acrylsäure verwendet, da die daraus gebildeten Polymere unter alkalischen Bedingungen hydrolysestabil sind. Es wurde gefunden, dass durch Pfropfpolymerisation derivatisierte Trennmaterialien mit erfindungsgemäß verbesserten Eigenschaften erhalten werden, wenn geeignete Trägermaterialien mit den in der folgenden Tabelle genannten Monomeren pfropfpolymerisiert werden.
2-Acrylamido-2-methylpropansulfonsäure (AMPS)
2-Acrylamidoethansulfonsäure Acrylsäure
2-(Acryloylaminoethyl)trimethylammoniumchlorid
2-(Diethylaminoethyl)acrylamid Dimethylacrylamid Methoxyethylacrylamid

Exemplarisch werden durch Kombination von zwei Monomeren die folgenden trägergebundenen Mischpfropfpolymere auf geeigneten Trägern, wie beispielsweise Fractogel TSK HW65 (M) oder (S) hergestellt und im Hinblick auf ihre Eigenschaften als Kationenaustauscher, insbesondere auf ihre Bindekapazität, untersucht:
Poly(AMPS, Dimethylacrylamid),
Poly(AMPS, Methoxyethylacrylamid)

Um Pfropfpolymere mit vorteilhaften Eigenschaften zu erhalten, werden geladene Monomere und ungeladene Monomere bevorzugt in einem solchen Verhältnis zueinander gemischt, dass der Anteil der geladenen Komponenten 1 - 90 mol-% im Verhältnis zur Gesamtmonomermenge beträgt, bevorzugt ist ein Anteil im Bereich von 3 - 70 mol-% bezogen auf die Gesamtmonomermenge. Zur Herstellung der erfindungsgemäßen Trennmaterialien werden die Monomere normalerweise im Überschuss zu dem Trägermaterial gegeben. Dabei werden 0,05 bis 100 mol Gesamtmonomer je Liter sedimentiertes Polymermaterial eingesetzt, bevorzugt werden 0,05 - 25 mol/L eingesetzt.

Es wurde gefunden, dass sich ein geladenes und ein ungeladenes Acrylamid im Gemisch pfropfpolymerisieren lassen und dass dabei die Einbauverhältnisse entsprechend der eingesetzten Monomerkonzentrationen erhalten werden (Tabelle 1).

Die aus den geladenen Monomeren und ungeladenen erfindungsgemäß hergestellten Pfropfpolymere liegen im wässerigen Milieu den pH-Werten der jeweiligen darin enthaltenen ionisierbaren Gruppen entsprechend und in Abhängigkeit vom aktuellen pH-Wert der wässerigen Umgebung mehr oder weniger stark ionisiert vor, in Form von Carboxylat- oder Sulfonatgruppen oder protonierten sekundären oder tertiären Aminogruppen oder quartären Ammoniumgrupen. Die erfindungsgemäßen Materialien eigenen sich daher besonders für die Anwendung in der Ionenaustauschchromatographie. Die ungeladenen Monomerbausteine sorgen dafür, dass der mittlere Abstand zwischen zwei Ladungen auf einem Pfropfpolymer größer ist als in einem Pfropfpolymer, das nur aus einem geladenen Monomerbaustein besteht. Da die Pfropfpolymere alle an eine Oberfläche geknüpft sind, bilden sie auf dieser ein Hydrogel, das dementsprechend durch die Anwesenheit von neutralen Gruppen eine geringere Ladungsdichte aufweist als ein Hydrogel, das nur aus einem geladenen Monomerbaustein besteht.

Zur Beurteilung der Kationenaustauscher wird zuerst die statische Bindungskapazität von Lysozym und polyclonalem human IgG (Gammanorm) im Bereich pH 5 - 7 untersucht. Die Bestimmung der Bindungskapazität erfolgt nach Elution des Proteins durch Erhöhen der Salzkonzentration auf ca. 1 M NaCl.

Die dynamische Bindungskapazität wird bei verschiedenen Flussraten gemessen, so dass bei Kontaktzeiten von 2 bzw. 8 min die in Säulen gepackten Kationenaustauscher mit Protein beladen wird. Dazu wird bis zu einem Durchbruch von 10% mit IgG (Gammanorm) in einem Puffer mit 25 mM Natriumphosphat und 25 mM Natriumacetat bei pH 5,0 gelöst beladen. Die Elution erfolgt durch Erhöhen der Salzkonzentration auf ca. 1 M NaCl bei dem pH des Bindungspuffers. Die Bindungskapazität wird aus der Proteinkonzentration (280 nM) im Eluat berechnet. Die Ergebnisse entsprechender Versuche sind in Tabelle 2 aufgeführt.

Überraschenderweise wurde gefunden, dass vergleichbare statische Bindungskapazitäten erreicht werden können, obwohl weniger geladenes Monomer für die Pfropfpolymerisation angeboten wird. So hat das als 08PP063 bezeichnete Produkt aus AMPS und Dimethylacrylamid sogar eine höhere statische Bindungskapazität für Lysozym und IgG im Vergleich zu dem Produkt 07PP221, obwohl für 08PP063 nur 66% der AMPS-Menge eingesetzt wurde, die für 07PP221 verwendet wurde.

Überraschenderweise wurde außerdem gefunden, dass die erfindungsgemäßen Trennmaterialien mit Mischpfropfpolymeren wesentlich höhere dynamische Bindungskapazitäten erzielen als Vergleichsgele. Dies gilt besonders für die Bindung von großen Molekülen, wie IgG, und bei hohen Flussraten (kurzen Kontaktzeiten) während der Beladung. Zum Beispiel erreicht das Mischpfropfpolymer 07SW261 aus AMPS und Methoxyethylacrylamid, in dem das molare Verhältnis von geladenem zu neutralen Monomerbausteinen ca. 1:0,9 beträgt (siehe Beispiele), bei pH 5,0 und einer Kontaktzeit von 2 min eine um 30% höhere Bindungskapazität als das kommerziell erhältlich Fractogel^{®} EMD SO₃ M.

Durch die neuartige Anordnung der negativen Ladungen im Raum über der Trägeroberfläche können insbesondere größere geladene Moleküle einfacher in das Hydrogel diffundieren. So trägt das IgG bei pH 5,0, bei dem die Bindung untersucht wurde, mehr positive als negative Ladungen (pH < pl). Möglichweise treten weniger abstoßende Wechselwirkungen der Pfropfpolymere mit den negativen Ladungen auf dem IgG statt. Durch erfindungsgemäßes Pfropfen auf porösen Trägern können also große Moleküle schneller von der äußeren Oberfläche des Trägers in das innere Porensystem transportiert und trotzdem für die Ionenaustauschchromatographie ausreichend fest gebunden werden.

Die erfindungsgemäßen Materialien können auch als mit Separationseffektoren versehene Polymere beschrieben werden. Sie können verwendet werden für die selektive, bedingt selektive oder unselektive Bindung oder Adsorption einer oder mehrerer Zielkomponenten mit dem Ziel der Abtrennung aus einer Matrix heraus, oder für die selektive, bedingt selektive oder unselektive Bindung oder Adsorption einer oder mehrerer Nebenkomponenten mit dem Ziel der Abtrennung der Nebenkomponente aus einer Matrix heraus, aber auch für die Auftrennung eines Stoffgemisches ohne Bindung oder Adsorption einer oder mehrerer Komponenten nur aufgrund der Molekulargröße gemäß Größenausschlusschromatographie, die Isolierung, Anreicherung und/oder Abreicherung von Biopolymeren aus natürlichen Quellen, die Isolierung, Anreicherung und/oder Abreicherung von Biopolymeren aus rekombinanten Quellen, die Isolierung, Anreicherung und/oder Abreicherung von Bioploymeren aus immortalisierten Zellinien sowie deren Kulturüberständen, die Isolierung, Anreicherung und/oder Abreicherung von Biopolymeren aus B-Zellinien und deren Derivaten, Lymphzellen und Hybridomazellinien und deren Kulturüberständen, die Isolierung, Anreicherung und/oder Abreicherung von Proteinen und Peptiden, die Isolierung, Anreicherung und/oder Abreicherung von Enzymen, die Isolierung, Anreicherung und/oder Abreicherung von monoklonalen und polyklonalen Antikörpern und natürlich vorkommenden oder rekombinanten Antikörperfragmenten, die Isolierung, Anreicherung und/oder Abreicherung phosphorylierter Peptide/Proteine und Nukleinsäuren, die Isolierung, Anreicherung und/oder Abreicherung von Lebensmittelzusatzstoffen, die Isolierung, Anreicherung und/oder Abreicherung von Mono- und Polysacchariden, die Isolierung, Anreicherung und/oder Abreicherung von glykosylierten Proteinen, die Isolierung, Anreicherung und/oder Abreicherung von einsträngiger oder doppelsträngiger DNA, die Isolierung, Anreicherung und/oder Abreicherung von Plasmid DNA, die Isolierung, Anreicherung und/oder Abreicherung von RNA, die Isolierung, Anreicherung und/oder Abreicherung von Viren, die Isolierung, Anreicherung und/oder Abreicherung von Host Cell Proteinen, die Isolierung, Anreicherung und/oder Abreicherung von Oligo- und Polynukleotiden, die Isolierung, Anreicherung und/oder Abreicherung von Liposomen, die Isolierung, Anreicherung und/oder Abreicherung von Produkten aus Blut und Milch, die Isolierung, Anreicherung und/oder Abreicherung von niedermolekularen Arzneimittelwirkstoffen (APIs: active pharmaceutical ingredient), der Abtrennung eines APIs von einem API-Arzneistoffträger (z.B. ein API-Liposom Addukt oder ein API-Nanopartikel Addukt), die Isolierung, Anreicherung und/oder Abreicherung von Enantiomeren

Die Biopolymere stammen vorwiegend, aber nicht ausschließlich, aus flüssigen Quellen oder sind in ihnen enthalten, wie zum Beispiel in Körperflüssigkeiten wie Blut, Seren, Speichel oder Urin, Organextrakten, Milch, Molke, Pflanzenextrakten, Zellextrakten, Zellkulturen, Fermentationsbrühen, tierischen Extrakten. Antikörper können zum Beispiel aus Säugerzellen von Nagetieren oder Hybridoma-Zellen stammen.

Die Zielmoleküle werden von mindestens einer oder mehreren anderen Substanzen aus einer Probe abgetrennt, wobei die Probe, die das Zielmolekül enthält, in einer Flüssigkeit gelöst ist, die mit dem erfindungsgemäßen Material in Kontakt gebracht wird. Kontaktzeiten sind üblicherweise im Bereich von 30 Sekunden bis zu 24 Stunden. Vorteilhaft ist es nach den Grundsätzen der Flüssigchromatographie zu arbeiten, indem die Flüssigkeit über eine Chromatographiesäule, die das erfindungsgemäße Trennmaterial enthält geleitet wird. Die Flüssigkeit kann allein durch ihre Schwerkraft durch die Säule laufen oder mit einer Pumpe hindurchgepumpt werden. Eine alternative Methode ist die Batch-Chromatographie. Hierbei wird das Trennmaterial mit der Flüssigkeit durch Rühren oder Schütteln so lange vermischt wie die Zielmoleküle, bzw. Biopolymere benötigen, um an das Trennmaterial binden zu können. Ebenfalls kann nach den Grundsätzen des chromatographischen Fliessbetts gearbeitet werden, indem die aufzutrennende Flüssigkeit beispielsweise in eine das Trennmaterial enthaltende Suspension gegeben wird, wobei das Trennmaterial so gewählt ist, dass es durch seine hohe Dichte und/oder einen magnetischen Kern für die gewünschte Abtrennung geeignet ist.

Das Zielmolekül bindet üblicherweise an das erfindungsgemäße Material. Anschließend kann das Trennmaterial mit einem Waschpuffer gewaschen werden, der vorzugsweise die gleiche Ionenstärke und den gleichen pH hat wie die Flüssigkeit, in der das Zielmolekül mit dem Trennmaterial in Kontakt gebracht wird. Der Waschpuffer entfernt alle Substanzen, die nicht an das Trennmaterial binden. Es können weitere Waschschritte mit anderen geeigneten Puffern folgen, ohne dass das Zielmolekül desorbiert. Die Desorption des gebundenen Zielmoleküls erfolgt durch Erhöhen der Ionenstärke im Eluenten oder durch Verändern des pH-Wertes im Eluenten. Das Zielmolekül kann so gereinigt und aufkonzentriert in dem Eluenten erhalten werden. Üblicherweise hat das Zielmolekül nach der Desorption eine Reinheit von 70% bis zu 99%, bevorzugt 85% bis 99%, besonders bevorzugt 90% - 99%.

Es besteht aber auch die Möglichkeit, dass das Zielmolekül in der Flüssigkeit verbleibt, andere Begleitsubstanzen aber an das Trennmaterial binden. Das Zielmolekül erhält man dann direkt durch Auffangen des Säuleneluats im Durchfluss. Dem Fachmann ist bekannt, wie er die Bedingungen, insbesondere den pH und/oder die Leitfähigkeit anpassen muss, um ein spezielles Biopolymer an ein Trennmaterial zu binden, oder ob es für die Reinigungsaufgabe von Vorteil ist, das Zielmolekül nicht zu binden.

Das erfindungsgemäße Trennmaterial kann in einem ersten chromatographischen Reinigungsschritt (Capture-Schritt) eines Aufarbeitungsprozesses für ein Biopolymer verwendet werden. Wenn der Capture-Schritt mit anderen Trennmaterialien erfolgt, kann es in einer der chromatographischen Reinigungsstufen, die dem Capture-Schritt folgen, eingesetzt werden, um die restlichen Verunreinigungen zu entfernen.

Die vorliegende Beschreibung ermöglicht es dem Fachmann die Erfindung umfassend anzuwenden. Auch ohne weitere Ausführungen wird daher davon ausgegangen, dass ein Fachmann die obige Beschreibung im weitesten Umfang nutzen kann.

Bei etwaigen Unklarheiten versteht es sich von selbst, die zitierten Veröffentlichungen und Patentliteratur heranzuziehen. Dementsprechend gelten diese Dokumente als Teil der Offenbarung der vorliegenden Beschreibung.

Zum besseren Verständnis und zur Verdeutlichung der Erfindung werden im folgenden Beispiele gegeben, die im Rahmen des Schutzbereichs der vorliegenden Erfindung liegen. Diese Beispiele dienen auch zur Veranschaulichung möglicher Varianten.

Weiterhin versteht es sich für den Fachmann von selbst, dass sich sowohl in den gegebenen Beispielen als auch in der übrigen Beschreibung die in den Zusammensetzungen enthaltenen Komponentenmengen in der Summe immer nur zu 100 Gew. bzw. mol-% bezogen auf die Gesamtzusammensetzung aufaddieren und nicht darüber hinausgehen können, auch wenn sich aus den angegebenen Prozentbereichen höhere Werte ergeben könnten. Sofern nichts anderes angegeben ist, gelten daher %-Angaben als Gew.- oder mol-%, mit Ausnahme von Verhältnissen, die in Volumenangaben wiedergegeben sind.

Die in den Beispielen und der Beschreibung sowie in den Ansprüchen gegebenen Temperaturen gelten immer in °C.

### Beispiele

### Vorschrift zur Herstellung eines Mischpfropfpolymers aus 2-Acrylamido-2-methylpropansulfonsäure und 2-Methoxyethylacrylamid (Ansatz 07SW261)

In einer Glasreaktionsapparatur mit einem Blattrührer wird 4,2 g Methoxyethylamin in 30 ml VE-Wasser auf 0 - 5 °C gekühlt. Unter kräftigem Rühren wird bei einer Innentemperatur von 0 - 5 °C über einen Tropftrichter 6,6 g 32%ige Natronlauge und aus einem zweiten Tropftrichter 4,8 g Acrylsäurechlorid zudosiert. Nach beendeter Zugabe wird die Lösung 30 min bei 5 °C nachgerührt und mit 65%iger Salpetersäure auf pH 6 gestellt und mit VE-Wasser auf 180 ml Gesamtvolumen aufgefüllt.

Aus dieser Lösung, 78 g nutschfeuchtem Fractogel TSK HW65 (M) (mit verdünnter Mineralsäure und VE-Wasser gewaschen) und einer Lösung aus 16,4 g 2-Acrylamido-2-methylpropansulfonsäure (AMPS) und 9,9 g 32%iger Natronlauge in 30 ml VE-Wasser wird eine Suspension hergestellt. Mit 32%iger Natronlauge und 65%iger Salpetersäure wird auf pH 6 gestellt.

Eine Starterlösung aus 1,6 g Ammoniumcer-IV-nitrat und 0,4 g 65%ige Salpetersäure in 40 ml VE-Wasser wird in einem Tropftrichter mit Druckausgleich vorgelegt. Die gesamte Apparatur wird durch wiederholtes (3x) Evakuieren und Entspannen mit Stickstoff inertisiert. Anschließend wird die Suspension in der Apparatur auf 40 °C erwärmt.

Zu der inertisierten Suspension wird bei einer Innentemperatur von 40 °C die Starterlösung unter Rühren zugegeben. Unter leichtem Stickstoffstrom wird die Suspension 18 Stunden bei 40 °C gerührt. Danach wird die Reaktionslösung über eine Glasfilterfritte (P2) abgesaugt und das Gel auf der Fritte mit jeweils 100 ml Waschlösung wie folgt gewaschen:
3x VE-Wasser
8x 1M Schwefelsäure, 0,2M Ascorbinsäure
3x VE-Wasser
2x 1M NaOH
3x VE-Wasser

Das Gel wird in 200 mL VE-Wasser suspendiert und mit 25%iger Salzsäure auf pH 7 eingestellt. Die Lagerung erfolgt in 20%igem Ethanol bei Raumtemperatur.

### Herstellung eines Mischpfropfpolymers aus 2-Acrylamido-2-methylpropansulfonsäure und Dimethylacrylamid (Ansatz 07PP221)

In einer Glasreaktionsapparatur mit einem Blattrührer wird eine Suspension aus 78 g nutschfeuchtem Fractogel TSK HW65 (M) (mit verdünnter Mineralsäure und VE-Wasser gewaschen) und einer Lösung von 5,1 g Dimethylacrylamid, 16,7 g 2-Acrylamido-2-methylpropansulfonsäure und 10,4 g 32%ige Natronlauge in 97 ml VE-Wasser hergestellt. Mit 32%iger Natronlauge und 65%iger Salpetersäure wird auf pH 6 gestellt und mit VE-Wasser auf 200 ml Gesamtvolumen aufgefüllt.

Eine Starterlösung aus 1,6 g Ammoniumcer-IV-nitrat und 0,4 g 65%ige Salpetersäure in 15 ml VE-Wasser wird in einem Tropftrichter mit Druckausgleich vorgelegt. Die gesamte Apparatur wird durch wiederholtes (3x) Evakuieren und Entspannen mit Stickstoff inertisiert. Anschließend wird die Suspension in der Apparatur auf 40 °C erwärmt.

Zu der inertisierten Suspension wird bei einer Innentemperatur von 40 °C die Starterlösung unter Rühren zugegeben. Unter leichtem Stickstoffstrom wird die Suspension 22 Stunden bei 40 °C gerührt. Danach wird die Reaktionslösung über eine Glasfilterfritte (P2) abgesaugt und das Gel auf der Fritte mit jeweils 100 ml Waschlösung wie folgt gewaschen:
7x VE-Wasser
10x 1M Schwefelsäure, 0,2M Ascorbinsäure
5x VE-Wasser
2x 1M Natronlauge
3x VE-Wasser
1x 50mM Phosphat-Puffer pH 7
2x VE-Wasser

Die Lagerung des Produktes erfolgt in 20%igem Ethanol bei Raumtemperatur.

### Herstellung eines Mischpfropfpolymers aus 2-Acrylamido-2-methylpropansulfonsäure und Dimethylacrylamid (Ansatz 08PP063)

In einer Glasreaktionsapparatur mit einem Blattrührer wird eine Suspension aus 100 g nutschfeuchtem Fractogel TSK HW65 (S) (mit verdünnter Mineralsäure und VE-Wasser gewaschen) und einer Lösung von 8,0 g Dimethylacrylamid, 11,1 g 2-Acrylamido-2-methylpropansulfonsäure und 6,9 g 32%ige Natronlauge in 60 ml VE-Wasser hergestellt. Mit 32%iger Natronlauge und 65%iger Salpetersäure wird auf pH 6,2 gestellt und mit VE-Wasser auf 190 ml Gesamtvolumen aufgefüllt.

Eine Starterlösung aus 1,6 g Ammoniumcer-IV-nitrat und 0,4 g 65%ige Salpetersäure in 25 ml VE-Wasser wird in einem Tropftrichter mit Druckausgleich vorgelegt. Die gesamte Apparatur wird durch wiederholtes (3x) Evakuieren und Entspannen mit Stickstoff inertisiert. Anschließend wird die Suspension in der Apparatur auf 40 °C erwärmt.

Zu der inertisierten Suspension wird bei einer Innentemperatur von 40 °C die Starterlösung unter Rühren zugegeben. Unter leichtem Stickstoffstrom wird die Suspension 22 Stunden bei 40 °C gerührt. Danach wird die Reaktionslösung über eine Glasfilterfritte (P2) abgesaugt und das Gel auf der Fritte mit jeweils 100 ml Waschlösung wie folgt gewaschen:
7x VE-Wasser
10x 1M Schwefelsäure, 0,2M Ascorbinsäure
5x VE-Wasser
2x 1M Natronlauge
3x VE-Wasser
1x 50mM Phosphat-Puffer pH 7,0
2x VE-Wasser
2x 20% Ethanol/150mM NaCl

Die Lagerung des Produktes erfolgt in 20%igem Ethanol bei Raumtemperatur.

### Bestimmung der chemischen Zusammensetzung der Pfropfpolymere

Aus den Pfropfpolymeren, bei denen es sich um Polyacrylamidketten handelt, können die funktionellen Gruppen durch saure Hydrolyse abgespalten werden. Die funktionellen Gruppen werden als Amin freigesetzt. Primäre Amine können nach Derivatisierung mit ortho-Phthaldialdehyd und Mercaptoethanol mit der HPLC quantitativ analysiert werden. Zur Kalibration werden käufliche Amine verwendet oder das in der Synthese verwendete Monomer, das dann wie das Pfropfpolymer hydrolisiert werden muss.

10 mg trockenes Gel werden mit 1000 µl 5 M Salzsäure versetzt, im Ultraschallbad beschallt und anschließend in einem 1 ml Druckbehälter 10 Stunden bei 125 °C erhitzt.

Der Druckbehälter wird nach Abkühlen auf Raumtemperatur geöffnet. Ca. 200 µl Überstand wird abpipettiert und 5 min zentrifugiert (8000 Upm).

40 µl des klaren Überstandes werden mit 176 µl 1 M Natronlauge neutralisiert und mit 325 µl 0,5 M Boratpuffer, pH 9,5 und 119 µl Acetonitril/Wasser 8:2 (V/V) versetzt und gemischt. Darauf wird 100 µl OPA-Reagenz, das aus 100 mg ortho-Phthaldialdehyd, 9 ml Methanol, 1 ml 0,5 M Boratpuffer pH 9,5 und 100 µl Mercaptoethanol hergestellt wird, zugesetzt und kräftig geschüttelt. Nach 2 Minuten Reaktionszeit wird die Probe mit einer HPLC analysiert (UV-Detektion 330 nm).

**Tabelle 1: Die Analyse des Pfropfpolymers von 07SW261 (Kalibration mit AMPS und Ethanolamin) im Vergleich zu den eingesetzten Monomermengen.**

| Baustein | Einsatz Monomer je g nutschfeuchtes Gel | Monomerbaustein je g trockenes gepfropftes Gel |
|---|---|---|
| AMPS | 1,0 mmol/g | 0,70 µmol/g |
| Methoxyethylacrylamid | 0,67 mmol/g* | 0,63 µmol/g |

| | | |
|---|---|---|
| *Einsatz Acrylsäurechlorid | | |

### Bestimmung der statischen IgG Bindungskapazität (Mikrotiterplattenformat)

Alle Gelsuspensionen werden mit 20% Ethanol in Wasser auf ein Gelsedimentvolumen von 50% eingestellt. Eine Filterplatte wird mit Bindepuffer und mit je 20 µl der homogenisierten Gelsuspension befüllt. Darauf wird die Filterplatte auf einer Vakuumstation absaugt.

Eine Deepwell-Platte wird mit Bindepuffer befüllt, mit Protein-Stammlösung (polyclonales human IgG Gammanorm, Fa. Octapharma oder Lysozym in Wasser) versetzt und gemischt, so dass die Konzentration 9,3 mg pIgG je ml bzw. 12,5 mg Lysozym je ml beträgt.

200 µl Protein-Lösung werden zum Gel in der Filterplatte gegeben und auf einem Schüttler 15 min geschüttelt. Die Filterplatte wird auf der Vakuumstation abgesaugt. Es wird zweimal mit je 100 µl Bindepuffer gewaschen und abgesaugt. Darauf werden je 200 µl Elutionspuffer (20 mM Phosphat, 1 M Natriumchlorid, pH 7) in die Filterplatte gegeben und 5 min geschüttelt. Der Überstand wird auf der Vakuumstation in eine UV-Platte gesaugt, die bei 280 nm im Photometer vermessen wird.

Die aus dem Eluat berechneten IgG Bindungskapazitäten pro 1 ml Gelsedimentvolumen (IgG SBC) sind in Tabelle 2 aufgelistet. Als Bindungspuffer werden 25 mM Natriumphosphat, 25 mM Natriumacetat, pH 5 für pIgG und 25 mM Natriumphosphat, 25 mM Natriumacetat, pH 7 für Lysozym verwendet.

### Bestimmung der dynamischen IgG Bindungskapazität

Es werden Säulen mit 1 ml Inhalt gepackt (Proteo-Cart-Säulen mit 19 mm Betthöhe und 20 % Kompression). Die Säulen werden mit einer IgG-Lösung mit einem Gehalt von 1 g/l in Puffer A (hergestellt aus polyclonalem human IgG Gammanorm, Fa. Octapharma) bis zu einem Durchbruch von 10% beladen. Dabei wird die Flußrate so gewählt, dass die Kontaktzeit auf der Säule 2 oder 8 min beträgt. Nach dem Nachspülen mit Puffer A, wird mit Puffer B eluiert.
Puffer A: 25 mM Natriumphosphat, 25 mM Natriumacetat, pH 5,0
Puffer B: 25 mM Natriumphosphat, 25 mM Natriumacetat, 1 M NaCl, pH 5,0

Die dynamische Bindungskapazität (DBC) von polyclonalem human IgG wird aus der photometrisch (280 nm) bestimmten Proteinmenge im Eluat berechnet und pro ml gepacktes Gel angegeben (Tabelle 2).

**Tabelle 2: Statische und dynamische Bindungskapazität (SBC und DBC) von polyclonalem human IgG (Gammanorm) bei pH 5,0 in mg Protein je ml sedimentiertes bzw. gepacktes Gel.**

| Ansatz | Lysozym SBC | IgG SBC | IgG DBC¹ | IgG DBC² |
|---|---|---|---|---|
| Fractogel EMD SO3 M | 104 | 124 | 70 | 131 |
| 07SW261 | 110 | 121 | 91 | 139 |
| 07PP221 | 86 | 126 | 82 | 138 |
| 08PP063 | 119 | 130 | 157 | 164 |

| | | | | |
|---|---|---|---|---|
| ¹ Kontaktzeit 2 min ² Kontaktzeit 8 min | | | | |

## Patentansprüche

1. Trennmaterialien für die Ionenaustauschchromatographie, enthaltend ein quervernetztes synthetisches Polymer oder eine quervernetzte Agarose oder ein quervernetztes Dextran
oder ein Kompositmaterial aus anorganischem Träger mit organischem Coating als Trägermaterial, auf dessen Oberfläche Mischpolymere kovalent gebunden sind, **dadurch gekennzeichnet, dass**
a) der Basisträger aliphatische Hydroxoylgruppen enthält,
b) die Mischpolymere kovalent an den Träger gebunden sind,
c) die Mischpolymere mindestens zwei verschiedene Monomereinheiten enthalten,
d) die Monomereinheiten linear verknüpft sind,
e) das Mischpolymer mindestens eine Monomereinheit aufweist, die eine Ladung in Form einer Sulfonsäure oder einer Carbonsäure trägt, oder eine Amin- oder Ammoniumgruppe trägt und Alkyl- und/oder Alkylengruppen und gegebenenfalls Amidgruppen enthält, jedoch keine Arylgruppen aufweist,
f) das Mischpolymer mindestens eine ungeladene Monomereinheit der allgemeinen Formel (1) 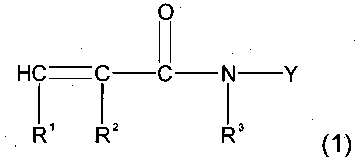 aufweist, worin
R¹ Wasserstoff,
R² Wasserstoff oder Methyl, und
R³ und Y unabhängig voneinander Wasserstoff, geradkettiges Alkyl mit bis zu 4 C-Atomen, Methoxypropyl, Ethoxyethyl oder Methoxyethyl bedeuten,
g) nur Monomereinheiten gleicher Ladung kombiniert sind, wenn mehr als ein Monomer mit Ladung enthalten ist,
h) das Verhältnis der Monomereinheiten mit Ladung zu den Monomereinheiten ohne Ladung in einem Bereich zwischen 1 : 99 bis 90 : 10 liegt.

2. Trennmaterialien für die Ionenaustauschchromatographie gemäß Anspruch 1, **dadurch gekennzeichnet, dass**
e) das Mischpolymer mindestens eine Monomereinheit mit Ladung der allgemeinen Formel (1') aufweist, 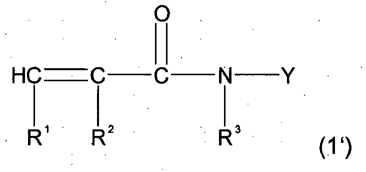 worin
R¹ Wasserstoff,
R² Wasserstoff oder Methyl, und
Y Wasserstoff und
R³ R⁴-SO₃Z, R⁴-COO, R⁴-NR⁹R¹⁰ oder R⁴-NR⁹R¹⁰R¹¹X
mit
R⁴ geradkettiges oder verzweigtes Alkylen mit 2 bis 4 C-Atomen,
R⁹, R¹⁰ und R¹¹ unabhängig von einander Wasserstoff, Methyl, Ethyl, Propyl, Phenyl oder Benzyl,
Z Wasserstoff, Na, K oder NH₄ und
X Cl, Br, J, oder Methylsulfat
bedeuten
oder das Mischpolymer mindestens eine Monomereinheit der allgemeinen Formel (2) 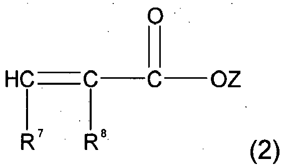 aufweist, worin
R⁷ Wasserstoff,
R⁸ Methyl,
Z Wasserstoff, Na, K, oder NH₄
bedeuten.

3. Trennmaterialien für die Ionenaustauscherchromatographie gemäß Anspruch 1, **dadurch gekennzeichnet, dass**
f) das Mischpolymer mindestens eine ungeladene Monomereinheit der allgemeinen Formel (1) aufweist, worin
R¹ Wasserstoff,
R² Wasserstoff oder Methyl und
R³ Methyl, Ethyl, Propyl, Methoxypropyl, Methoxyethyl mit
Y Wasserstoff oder
R³ Methyl oder Ethyl mit
Y Methyl
bedeuten.

4. Trennmaterialien nach einem oder mehreren der Ansprüche 1 bis 3, **dadurch gekennzeichnet dass**
e) das Mischpolymer mindestens eine Monomereinheit mit negativer Ladung ausgewählt aus der Gruppe 2-Acrylamido-2-methylpropan-sulfonsäure, 2-Acrylamidoethansulfonsäure, Carboxymethylacrylamid; Carboxyethylacrylamid, Carboxypropylacrylamid, Carboxymethlymethacrylamid, Carboxyethylmethacrylamid, Carboxypropylmethacrylamid, Acrylsäure und Methacrylsäure, aufweist
und
f) das Mischpolymer mindestens eine ungeladene Monomereinheit gemäß der allgemeinen Formel (1),
ausgewählt aus der Gruppe N,N-(Dimethyl)acrylamid, N-(Ethoxyethyl)acrylamid, N-(Methoxyethyl)acrylamid und N-(Methoxypropyl)-acrylamid, enthält.

5. Trennmaterialien nach einem oder mehreren der Ansprüche 1 bis 4, **dadurch gekennzeichnet dass**
e) das Mischpolymer mindestens eine Monomereinheit mit positiver Ladung, ausgewählt aus der Gruppe 2-(Acryloylaminoethyl)trimethylammoniumchlorid, 3-(Acryloylaminopropyl)-trimethylammoniumchlorid, 2-(Diethylaminoethyl)acrylamid, 2-(Diethylaminoethyl)methacrylamid, 2-(Dimethylaminoethyl)acrylamid, 2-(Dimethylaminoethyl)-methacrylamid, 3-(Diethylaminopropyl)acrylamid, 3-(Diethylaminopropyl)methacrylamid, 3-(Diethylaminopropyl)acrylamid, 3-(Diethylaminopropyl)methacrylamid, 2-(Methacryloylaminoethyl)-trimethylammoniumchlorid und 3-(Acryloyl-aminopropyl)-trimethylammoniumchlorid aufweist
und
f) das Mischpolymer mindestens eine ungeladene Monomereinheit gemäß der allgemeinen Formel (1),
ausgewählt aus der Gruppe N,N-(Dimethyl)acrylamid, N-(Ethoxyethyl)acrylamid, N-(Methoxyethyl)acrylamid und N-(Methoxypropyl)-acrylamid, enthält.

6. Verfahren zur Herstellung von Trennmaterialien gemäß einem oder mehreren der Ansprüche 1 bis 5, **dadurch gekennzeichnet, dass** Mischpolymere an Oberflächen eines Basisträgers mit aliphatischen Hydroxylgruppen,
wobei es sich um Oberflächen von einem quervernetzten synthetischen Polymer oder einer quervernetzten Agarose oder einem quervernetzten Dextran oder einem Kompositmaterial aus anorganischem Träger mit organischem Coating handelt,
als Trägermaterial gebunden werden,
indem
durch eine einstufige Polymerisationsreaktion,
welche durch Cer(IV)-lonen auf dem hydroxylgruppen-haltigen Träger initiiert wird,
Pfropfpolymere erhalten werden,
die kovalent an die hydroxylgruppenhaltige Oberfläche gebunden sind, und
die aus zwei oder mehreren wiederkehrenden Monomereinheiten aufgebaut sind und die linear verknüpft sind,
wobei mindestens eine dieser Monomereinheiten eine Ladung
in Form einer Sulfonsäure oder einer Carbonsäure trägt, oder
eine Amin- oder Ammoniumgruppe trägt und Alkyl- und/oder Alkylengruppen und gegebenenfalls Amidgruppen enthält, jedoch keine Arylgruppen aufweist,
und mindestens eine dieser wiederkehrenden Monomereinheiten ungeladen ist und die allgemeinen Formel (1) 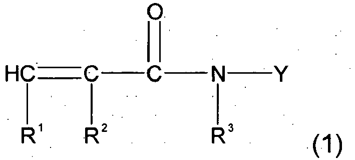 aufweist, worin
R¹ Wasserstoff,
R² Wasserstoff oder Methyl, und
R³ und Y unabhängig voneinander Wasserstoff, geradkettiges Alkyl mit bis zu 4 C-Atomen, Methoxypropyl, Ethoxyethyl oder Methoxyethyl bedeuten,
wobei nur Monomereinheiten gleicher Ladung kombiniert sind, wenn mehr als ein Monomer mit Ladung im Pfropfpolymer enthalten ist,
und das Verhältnis der Monomereinheiten mit Ladung zu den Monomereinheiten ohne Ladung in einem Bereich zwischen
1 : 99 bis 90 : 10 liegt,
wodurch Materialien erhalten werden, die durch ionische Wechselwirkung geladene Substanzen, insbesondere Biopolymere, zu binden vermögen.

## Claims

1. Separating materials for ion exchange chromatography, comprising a crosslinked synthetic polymer or a crosslinked agarose or a crosslinked dextran
or a composite material comprising an inorganic support with an organic coating as support material, to the surface of which copolymers are covalently bonded, **characterised in that**
a) the base support contains aliphatic hydroxyl groups,
b) the copolymers are covalently bonded to the support,
c) the copolymers contain at least two different monomer units,
d) the monomer units are linked in a linear manner,
e) the copolymer has at least one monomer unit which carries a charge in the form of a sulfonic acid or carboxylic acid, or carries an amine or ammonium group and contains alkyl and/or alkylene groups and optionally amide groups, but no aryl groups,
f) the copolymer has at least one uncharged monomer unit of the general formula (1) 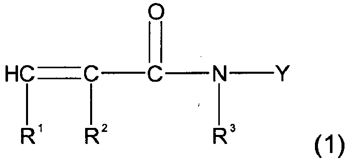 in which
R¹ denotes hydrogen,
R² denotes hydrogen or methyl, and
R³ and Y, independently of one another, denote hydrogen, straight-chain alkyl having up to 4 C atoms, methoxypropyl, ethoxyethyl or methoxyethyl,
g) only monomer units of the same charge are combined if more than one monomer having a charge is present,
h) the ratio of the monomer units having a charge to the monomer units without a charge is in a range between 1 : 99 to 90 : 10.

2. Separating materials for ion exchange chromatography according to Claim 1, **characterised in that**
e) the copolymer has at least one monomer unit having a charge of the general formula (1'), 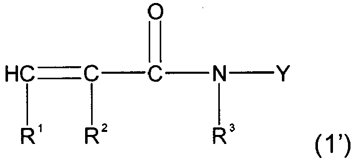 in which
R¹ denotes hydrogen,
R² denotes hydrogen or methyl, and
Y denotes hydrogen and
R³ denotes R⁴-SO₃Z, R⁴-COO, R⁴-NR⁹R¹⁰ or R⁴-NR⁹R¹⁰R¹¹X
where
R⁴ denotes straight-chain or branched alkylene having 2 to 4 C atoms,
R⁹, R¹⁰ and R¹¹, independently of one another, denote hydrogen, methyl, ethyl, propyl, phenyl or benzyl,
Z denotes hydrogen, Na, K or NH₄ and
X denotes Cl, Br, I or methylsulfate
or the copolymer has at least one monomer unit of the general formula (2) 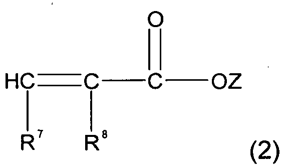 in which
R⁷ denotes hydrogen,
R⁸ denotes methyl,
Z denotes hydrogen, Na, K or NH₄.

3. Separating materials for ion exchange chromatography according to Claim 1, **characterised in that**
f) the copolymer has at least one uncharged monomer unit of the general formula (1), in which
R¹ denotes hydrogen,
R² denotes hydrogen or methyl and
R³ denotes methyl, ethyl, propyl, methoxypropyl, methoxyethyl where
Y denotes hydrogen or
R³ denotes methyl or ethyl where
Y denotes methyl.

4. Separating materials according to one or more of Claims 1 to 3, **characterised in that**
e) the copolymer has at least one monomer unit having a negative charge, selected from the group 2-acrylamido-2-methylpropanesulfonic acid, 2-acrylamidoethanesulfonic acid, carboxymethylacrylamide, carboxyethylacrylamide, carboxypropylacrylamide, carboxymethlymethacrylamide, carboxyethylmethacrylamide, carboxypropylmethacrylamide, acrylic acid and methacrylic acid,
and
f) the copolymer contains at least one uncharged monomer unit of the general formula (1),
selected from the group N,N-(dimethyl)acrylamide, N-(ethoxyethyl)-acrylamide, N-(methoxyethyl)acrylamide and N-(methoxypropyl)-acrylamide.

5. Separating materials according to one or more of Claims 1 to 4, **characterised in that**
e) the copolymer has at least one monomer unit having a positive charge, selected from the group 2-(acryloylaminoethyl)trimethylammonium chloride, 3-(acryloylaminopropyl)trimethylammonium chloride, 2-(diethylaminoethyl)acrylamide, 2-(diethylaminoethyl)-methacrylamide, 2-(dimethylaminoethyl)acrylamide, 2-(dimethylaminoethyl)methacrylamide, 3-(diethylaminopropyl)acrylamide, 3-(diethylaminopropyl)methacrylamide, 3-(diethylaminopropyl)acrylamide, 3-(diethylaminopropyl)methacrylamide, 2-(methacryloylaminoethyl)-trimethylammonium chloride and 3-(acryloylaminopropyl)trimethylammonium chloride
and
f) the copolymer contains at least one uncharged monomer unit of the general formula (1),
selected from the group N,N-(dimethyl)acrylamide,N-(ethoxyethyl)-acrylamide, N-(methoxyethyl)acrylamide and N-(methoxypropyl)acrylamide.

6. Process for the preparation of separating materials according to one or more of Claims 1 to 5, **characterised in that** copolymers are bonded to surfaces of a base support having aliphatic hydroxyl groups,
where the surfaces are of a crosslinked synthetic polymer or a crosslinked agarose or a crosslinked dextran or a composite material comprising inorganic support with organic coating,
as support material,
in which a one-step polymerisation reaction,
which is initiated by cerium(IV) ions on the hydroxyl-containing support, gives graft polymers
which are covalently bonded to the hydroxyl-containing surface
and
which are built up from two or more recurring monomer units and which are linked in a linear manner,
where at least one of these monomer units carries a charge in the form of a sulfonic acid or carboxylic acid, or carries an amine or ammonium group and contains alkyl and/or alkylene groups and optionally amide groups, but no aryl groups,
and at least one of these recurring monomer units is uncharged and
has the general formula (1) 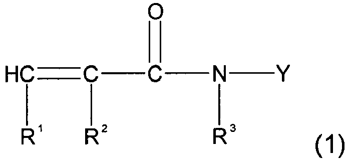 in which
R¹ denotes hydrogen,
R² denotes hydrogen or methyl, and
R³ and Y, independently of one another, denote hydrogen, straight-chain alkyl having up to 4 C atoms, methoxypropyl, ethoxyethyl or methoxyethyl,
where only monomer units of the same charge are combined if more than one monomer having a charge is present in the graft polymer, and the ratio of the monomer units having a charge to the monomer units without a charge is in a range between 1 : 99 to 90 : 10, giving materials which are capable of binding charged substances, in particular biopolymers, by ionic interaction.

## Revendications

1. Matériaux de séparation pour chromatographie d'échange d'ions, comprenant un polymère synthétique réticulé ou un agarose réticulé ou un dextrane réticulé
ou un matériau composite comprenant un support inorganique ayant un revêtement organique comme matériau support, à la surface duquel les copolymères sont liés de manière covalente, **caractérisés en ce que**
a) le support de base contient des groupements hydroxyle aliphatiques,
b) les copolymères sont liés de manière covalente au support,
c) les copolymères contiennent au moins deux motifs monomères différents,
d) les motifs monomères sont liés de manière linéaire,
e) le copolymère possède au moins un motif monomère qui porte une charge sous la forme d'un acide sulfonique ou d'un acide carboxylique, ou porte un groupement amine ou ammonium et contient des groupements alkyle et/ou alkylène et éventuellement des groupements amide, mais aucun groupement aryle,
f) le copolymère possède au moins un motif monomère non chargé de formule générale (1) 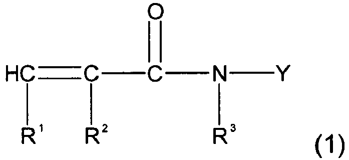 dans laquelle
R¹ désigne hydrogène,
R² désigne hydrogène ou méthyle, et
R³ et Y, indépendamment l'un de l'autre, désignent hydrogène, alkyle à chaîne linéaire ayant jusqu'à 4 atomes de C, méthoxypropyle, éthoxyéthyle ou méthoxyéthyle,
g) seuls des motifs monomères de même charge sont combinés si plus d'un monomère ayant une charge est présent,
h) le rapport des motifs monomères ayant une charge aux motifs monomères sans charge se trouve dans une plage allant de 1:99 à 90:10.

2. Matériaux de séparation pour chromatographie d'échange d'ions selon la revendication 1, **caractérisés en ce que**
e) le copolymère possède au moins un motif monomère ayant une charge de formule générale (1'), 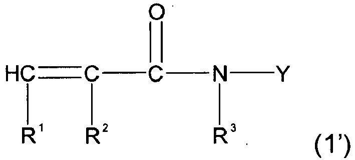 dans laquelle
R¹ désigne hydrogène,
R² désigne hydrogène ou méthyle, et
Y désigne hydrogène et
R³ désigne R⁴-SO₃Z, R⁴-COO, R⁴-NR⁹R¹⁰ ou R⁴-NR⁹R¹⁰R¹¹X
où
R⁴ désigne alkylène à chaîne linéaire ou ramifiée ayant de 2 à 4 atomes de C,
R⁹, R¹⁰ et R¹¹, indépendamment l'un de l'autre, désignent hydrogène, méthyle, éthyle, propyle, phényle ou benzyle,
Z désigne hydrogène, Na, K ou NH₄ et
X désigne Cl, Br, I ou méthylsulfate
ou le copolymère possède au moins un motif monomère de formule générale (2) 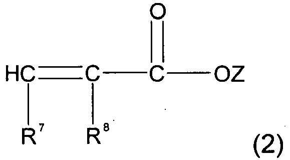 dans laquelle
R⁷ désigne hydrogène,
R⁸ désigne méthyle,
Z désigne hydrogène, Na, K ou NH₄.

3. Matériaux de séparation pour chromatographie d'échange d'ions selon la revendication 1, **caractérisés en ce que**
f) le copolymère possède au moins un motif monomère non chargé de formule générale (1), dans laquelle
R¹ désigne hydrogène,
R² désigne hydrogène ou méthyle et
R³ désigne méthyle, éthyle, propyle, méthoxypropyle, méthoxyéthyle où
Y désigne hydrogène ou
R³ désigne méthyle ou éthyle où
Y désigne méthyle.

4. Matériaux de séparation selon l'une ou plusieurs parmi les revendications 1 à 3, **caractérisés en ce que**
e) le copolymère possède au moins un motif monomère ayant une charge négative, choisi dans le groupe constitué par l'acide 2-acryl-amido-2-méthylpropanesulfonique, l'acide 2-acrylamidoéthane-sulfonique, le carboxyméthylacrylamide, le carboxyéthylacrylamide, le carboxypropylacrylamide, le carboxyméthlyméthacrylamide, le carboxyéthylméthacrylamide, le carboxypropylméthacrylamide,
l'acide acrylique et l'acide méthacrylique,
et
f) le copolymère contient au moins un motif monomère non chargé de formule générale (1),
choisi dans le groupe constitué par le N,N-(diméthyl)acrylamide, le N-(éthoxyéthyl)acrylamide, le N-(méthoxyéthyl)acrylamide et le N-(méthoxypropyl)acrylamide.

5. Matériaux de séparation selon l'une ou plusieurs parmi les revendications 1 à 4, **caractérisés en ce que**
e) le copolymère possède au moins un motif monomère ayant une charge positive, choisi dans le groupe constitué par le chlorure de 2-(acryloylaminoéthyl)triméthylammonium, le chlorure de 3-(acryloyl-aminopropyl)triméthylammonium, le 2-(diéthylaminoéthyl)acrylamide, le 2-(diéthylaminoéthyl)méthacrylamide, le 2-(diméthylaminoéthyl)-acrylamide, le 2-(diméthylaminoéthyl)méthacrylamide, le 3-(diéthyl-aminopropyl)acrylamide, le 3-(diéthylaminopropyl)méthacrylamide, le 3-(diéthylaminopropyl)acrylamide, le 3-(diéthylaminopropyl)méthacryl-amide, le chlorure de 2-(méthacryloylaminoéthyl)triméthylammonium et le chlorure de 3-(acryloylaminopropyl)triméthylammonium
et
f) le copolymère contient au moins un motif monomère non chargé de formule générale (1),
choisi dans le groupe constitué par le N,N-(diméthyl)acrylamide, le N-(éthoxyéthyl)acrylamide, le N-(méthoxyéthyl)acrylamide et le N-(méthoxypropyl)acrylamide.

6. Procédé de préparation de matériaux de séparation selon l'une ou plusieurs parmi les revendications 1 à 5, **caractérisé en ce que** des copolymères sont fixés à des surfaces d'un support de base ayant des groupements hydroxyle aliphatiques,
où les surfaces sont constituées d'un polymère synthétique réticulé ou d'un agarose réticulé ou d'un dextrane réticulé ou d'un matériau composite comprenant un support inorganique ayant un revêtement organique,
comme matériau support,
où une réaction de polymérisation à une étape,
qui est initiée par des ions cérium(IV) sur le support contenant des groupements hydroxyle,
produit des polymères greffés
qui sont liés de manière covalente à la surface contenant des groupements hydroxyle
et
qui sont bâtis à partir de deux, ou plus, motifs monomères récurrents et qui sont liés de manière linéaire,
où au moins l'un de ces motifs monomères porte une charge sous la forme d'un acide sulfonique ou d'un acide carboxylique, ou porte un groupement amine ou ammonium et contient des groupements alkyle et/ou alkylène et éventuellement des groupements amide, mais aucun groupement aryle,
et au moins l'un de ces motifs monomères récurrents est non chargé et répond à la formule générale (1) 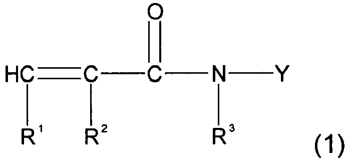 dans laquelle
R¹ désigne hydrogène,
R² désigne hydrogène ou méthyle, et
R³ et Y, indépendamment l'un de l'autre, désignent hydrogène, alkyle à chaîne linéaire ayant jusqu'à 4 atomes de C, méthoxypropyle, éthoxyéthyle ou méthoxyéthyle,
où seuls des motifs monomères de même charge sont combinés si plus d'un monomère ayant une charge est présent dans le polymère greffé, et le rapport des motifs monomères ayant une charge aux motifs monomères sans charge se trouve dans une plage allant de 1:99 à 90:10,
pour conduire à des matériaux qui sont capables de fixer des substances chargées, en particulier des biopolymères, par interaction ionique.
